# FASCICULE DE BREVET EUROPEEN

(11) **EP 0 892 776 B1**
(45) Date de publication et mention de la délivrance du brevet: **12.12.2001**
(21) Numéro de dépôt: 97919429.7
(22) Date de dépôt: 27.03.1997
(51) Int. Cl.: C07C 53/08, C07C 51/353, C07C 69/14, C07C 67/293

(54) **PROCEDE DE PREPARATION D'ACIDE ACETIQUE ET/OU D'ACETATE DE METHYLE PAR ISOMERISATION DE FORMIATE DE METHYLE**
VERFAHREN ZUR HERSTELLUNG DER ESSIGSÄURE UND/ODER METHYLACETAT DURCH ISOMERISIERUNG DES METHYLFORMIATS
METHOD FOR PREPARING ACETIC ACID AND/OR METHYL ACETATE BY METHYL FORMATE ISOMERISATION

(30) Priorité: 27.03.1996 FR 9603781
(43) Date de publication de la demande: 27.01.1999
(73) Titulaire: Acetex Chimie, 92200 Neuilly-sur-Seine (FR)
(72) Inventeur: PATOIS, Carl, F-68400 Riedisheim (FR); PERRON, Robert, F-69390 Charly (FR); THIEBAUT, Daniel, F-64140 Billère (FR)
(74) Mandataire: Giraud, Françoise
(86) Numéro de dépôt international: FR9700553
(87) Numéro de publication internationale: WO9735829

(56) Documents cités:
- EP-A- 0 045 637
- DE-A- 3 046 899
- US-A- 4 613 693

## Description

La présente invention a pour objet la préparation d'acide acétique et/ou d'acétate de méthyle, en mettant en oeuvre une réaction d'isomérisation de formiate de méthyle.

Diverses voies d'accès à l'acide acétique sont connues et exploitées industriellement. Parmi celles-ci figurent les réactions de carbonylation du méthanol. Cette réaction de carbonylation peut notamment être mise en oeuvre en phase liquide, sous pression de monoxyde de carbone, qui est l'un des réactifs, en présence d'un système catalytique homogène comprenant un composé à base de rhodium et/ou d'iridium et un promoteur iodé.

Une autre voie d'accès est constituée par la réaction d'isomérisation de formiate de méthyle en présence d'un catalyseur à base de rhodium ou d'iridium.

Dans les procédés d'isomérisation catalysée avec de l'iridium, la réaction est effectuée avec un solvant qui est choisi parmi les acides carboxyliques, et plus particulièrement, l'acide produit. La réaction est par ailleurs mise en oeuvre sous une atmosphère comprenant de l'azote. II a en effet été constaté que le monoxyde de carbone durant cette réaction n'apportait pas d'avantage particulier et pouvait même être la cause d'une certaine inhibition de la réaction d'isomérisation, favorisant les réactions secondaires. Il est à noter qu'un tel comportement est tout à fait différent de celui observé lorsque le système catalytique est à base de rhodium, cas pour lequel la présence de monoxyde de carbone est essentielle au maintien en phase homogène du métal. Ce type de procédé dont l'intérêt n'est pas remis ici en cause ne présente pas un véritable intérêt sur le plan industriel car la réaction qui y est décrite n'est pas suffisamment performante. En effet, les vitesses de réaction sont seulement de l'ordre de 2 mol/h/l d'acide et/ou d'ester produits.

Il a été proposé, afin d'améliorer les résultats du procédé précité, de mettre en oeuvre la réaction d'isomérisation en présence d'un acide fort du type des acides sulfoniques, comme l'acide paratoluène sulfonique par exemple. Dans les conditions de ce procédé, la réaction est effectuée en mettant en oeuvre des quantités importantes de formiate de méthyle à isomériser, qui est par conséquent aussi utilisé comme solvant de la réaction. Si ce perfectionnement contribue à augmenter l'activité de la réaction, il présente cependant l'inconvénient de nécessiter l'emploi d'un composé supplémentaire, ce qui ne simplifie pas le procédé.

En outre, il est possible que cet acide se dégrade dans les conditions du milieu réactionnel.

La présente invention a pour but de proposer un procédé d'isomérisation de formiate de méthyle, en acide acétique et/ou acétate de méthyle, dont la productivité est améliorée par rapport aux deux variantes d'isomérisation précédemment décrites, ceci sans qu'il soit nécessaire d'employer de composé supplémentaire dans cet objectif,

Par ailleurs, le procédé selon l'invention est très sélectif.

Ces buts et d'autres sont atteints par la présente invention qui a donc pour objet la préparation d'acide acétique et/ou d'acétate méthyle, par réaction d'isomérisation du formiate de méthyle, en présence d'eau, d'un solvant et d'un système catalytique comprenant au moins un promoteur halogéné et au moins un composé à base d'iridium, caractérisé en ce que ledit procédé est un procédé continu dans lequel on maintient une pression partielle en monoxyde de carbone comprise entre 0,1.10⁵ Pa et 25.10⁵ Pa, une quantité de formiate de méthyle inférieure à 20 % en poids du mélange réactionnel et une quantité d'eau comprise entre 0 exclu et 5% en poids du mélange réactionnel.

On a en effet constaté que contrairement à ce qui était prétendu dans l'art antérieur, la présence de monoxyde de carbone était essentielle pour la réaction d'isomérisation de l'ester en présence d'iridium.

Par ailleurs, une autre caractéristique importante de la réaction est que la quantité de formiate de méthyle réagissant doit être d'au plus 20 % pour obtenir les meilleures productivités.

Ainsi, la combinaison de ces deux caractéristiques a permis de multiplier par 10 la productivité du premier procédé décrit, simplement en maintenant durant la réaction, les conditions de pression partielle en monoxyde de carbone et de concentration en ester dans les intervalles mentionnés. Par ailleurs, la productivité du procédé selon l'invention est meilleure que celle de la variante comprenant l'acide fort car des vitesses de réaction du même ordre de grandeur, voire supérieures, ont été obtenues à des températures plus faibles.

Pour plus de clarté, la nature des réactifs va tout d'abord être décrite.

Ainsi, la réaction est mise en oeuvre avec le formiate de méthyle.

Le procédé de l'invention est mis en oeuvre en présence d'un système catalytique comprenant au moins un promoteur halogéné et au moins un composé à base d'iridium.

Le promoteur halogéné, représentant l'un des constituants du système catalytique, est choisi de préférence parmi les composés iodés.

Le promoteur halogéné peut se présenter sous la forme d'iode seul, ou en combinaison avec d'autres éléments comme par exemple l'hydrogène, un radical alkyle en C₁-C₁₀, un radical acyle en C₁-C₁₀, un radical aryle en C₆-C₁₀., ou encore des iodures de métal alcalin ou des iodures métalliques, tels que les iodures de métaux de transition, ou de la colonne IIA de la classification périodique des éléments.

Il est à noter que le promoteur halogéné peut être constitué d'un mélange de plusieurs des promoteurs précités.

On ne sortirait pas du cadre de la présente invention en préparant in situ lesdits promoteurs halogénés, à l'aide de précurseurs appropriés.

A titre d'exemple de promoteurs convenant à la présente invention, on peut notamment citer, sans intention de se limiter, l'iode, l'acide iodhydrique, l'iodure de méthyle, l'iodure d'éthyle, le diiodure-1,1 d'éthyle, l'iodure d'acétyle, l'iodure d'aluminium, l'iodure de chrome, l'iodure de lithium, l'iodure de potassium.

Selon un mode de réalisation particulier de l'invention, le promoteur mis en oeuvre comprend l'hydrogène ou un radical alkyle en C₁-C₁₀. De préférence, le promoteur halogéné comprend l'iode et un radical du type méthyle.

Le second élément du système catalytique mis en oeuvre dans le procédé selon l'invention est constitué par au moins un composé à base d'iridium.

Tout d'abord, la réaction selon l'invention est plus particulièrement mise en oeuvre en présence d'un catalyseur homogène. En d'autres termes, cela signifie que le composé à base d'iridium notamment se trouve sous une forme soluble dans le mélange réactionnel. II est à noter que la présence d'une partie dudit composé à base d'iridium sous une forme non solubilisée, ne présente pas de difficulté majeure pour la mise en oeuvre de la réaction.

Tous les composés de l'iridium solubles ou pouvant être solubilisés dans le milieu réactionnel, dans les conditions de réalisation de l'invention, peuvent être utilisés. A titre d'exemple et sans intention de se limiter, conviennent notamment à la mise en oeuvre de l'invention, l'iridium à l'état métallique, les sels simples de ce métal, les oxydes ou encore les complexes de coordination.

En tant que sels simples d'iridium, on utilise habituellement les halogénures d'iridium. L'halogène est plus particulièrement choisi parmi le chlore, le brome ou l'iode, ce dernier étant préféré. Ainsi les composés comme Irl₃, lrBr₃, IrCl₃, Irl₃.4H₂O, Irl₄, IrBr₃.4H₂O peuvent être utilisés dans le procédé selon l'invention.

Les oxydes choisis parmi IrO₂, Ir₂O₃. xH₂O peuvent de même être convenablement mis en oeuvre dans le procédé selon l'invention.

En ce qui concerne les complexes solubles de coordination de l'iridium, les composés les plus couramment mis en oeuvre sont ceux présentant des ligands choisis parmi le monoxyde de carbone ou une combinaison monoxyde de carbone/halogène, l'halogène étant choisi parmi le chlore, le brome ou plus particulièrement l'iode. II n'est toutefois pas exclu d'utiliser des complexes solubles d'iridium dont les ligands sont choisis parmi des composés organo phosphorés ou organo azotés, par exemple.

En tant que complexes de coordination, connus de l'homme du métier, convenant particulièrement à la mise en oeuvre de l'invention, on peut citer sans intention de se limiter les composés suivants : Ir₄(CO)₁₂, Ir(CO)₂I₂⁻Q⁺, Ir(CO)₂Br₂⁻Q⁺, Ir(CO)₂Cl₂⁻Q⁺ ; formules dans lesquelles Q peut représenter notamment l'hydrogène, le groupe NR₄, PR₄, avec R choisi parmi l'hydrogène, un radical hydrocarboné.

Ces catalyseurs peuvent être obtenus par toute méthode connue de l'homme du métier. Ainsi, on pourra se reporter aux demandes de brevets EP 657 386 et WO95/17963 pour la préparation de solutions catalytiques à base d'iridium convenant à la réalisation de la présente invention.

II est à noter que la réaction selon l'invention peut être mise en oeuvre avec un système catalytique comprenant, outre l'iridium, un ou plusieurs autres métaux du groupe VIII. Plus particulièrement, la réaction peut être mise en oeuvre avec une association de rhodium et d'iridium, ou encore une association de ruthénium et d'iridium, ou encore un système catalytique à base de ces trois métaux.

Si une telle variante est adoptée, le rapport molaire de l'iridium aux autres métaux associés est plus particulièrement compris entre 1/10 et 10/1. De préférence, il est supérieur à 1/1.

Comme cela a été mentionné auparavant, la réaction d'isomérisation selon l'invention est mise en oeuvre en présence d'eau et d'un solvant.

En ce qui concerne le solvant, celui-ci peut comprendre un ou plusieurs acides carboxyliques, ainsi que d'autres composés dénommés co-solvants au titre de la présente invention.

Plus particulièrement ledit solvant est choisi parmi les acides carboxyliques. Selon un mode de réalisation particulier de la présente invention, l'acide carboxylique est choisi parmi les acides aliphatiques présentant de 2 à 10 atomes de carbone, de préférence comprenant de 2 à 5 atomes de carbone. Selon un mode de réalisation particulièrement avantageux de la présente invention, ledit acide carboxylique est l'acide acétique. On ne sortirait pas du cadre de la présente invention en employant un mélange d'acides précités.

La réaction selon l'invention est par ailleurs mise en oeuvre en présence d'acide formique, présent dans le milieu et, par conséquent, compté parmi les acides carboxyliques. L'acide formique fait également partie des solvants intervenant dans la réaction mise en oeuvre.

Bien entendu, on ne sortirait pas du cadre de la présente invention en mettant en oeuvre un solvant supplémentaire, inerte dans les conditions de réaction. A titre d'exemple de ce type de solvant, on peut citer les esters, les éthers, les cétones, les amides, les sulfoxides ou encore les hydrocarbures. Le co-solvant préféré est l'acétate de méthyle.

Si des co-solvants sont employés, la quantité d'acide carboxylique est de préférence supérieure à celle du co-solvant.

Le procédé de l'invention consiste donc à maintenir pendant la durée de la réaction, une pression partielle en monoxyde de carbone et une concentration en formiate de méthyle spécifiques.

Ainsi, la pression partielle en monoxyde de carbone est maintenue comprise entre 0,1.10⁵ Pa et 25.10⁵ Pa.

Les pressions sont exprimées en pascals absolus, et ont été mesurées à chaud, c'est-à-dire dans les conditions de température de la réaction.

Selon un mode de réalisation plus particulier de l'invention, on maintient une pression partielle en monoxyde de carbone supérieure à 0,5.10⁵ Pa et de préférence supérieure à 10⁵ Pa.

La pression partielle en monoxyde de carbone de manière avantageuse est inférieure 15.10⁵ Pa. Plus particulièrement, elle est inférieure à 10.10⁵ Pa.

Une seconde caractéristique importante de la présente invention est que la teneur en formiate de méthyle est maintenue inférieure à 20 % en poids du mélange réactionnel.

De manière préférée, ladite teneur en ester précité ne dépasse pas 10 % en poids du mélange réactionnel. Selon un mode de réalisation particulièrement avantageux de la présente invention, la teneur en formiate de méthyle ne dépasse pas 5 % en poids du mélange réactionnel.

Lorsque la réaction est mise en oeuvre en continu, les caractéristiques précitées sont de préférence maintenues constantes pendant le cours de la réaction. Il est à noter que la pression partielle en monoxyde de carbone peut évoluer pendant la réaction, dans la mesure où elle se trouve toujours comprise dans la gamme précitée.

Lorsque la réaction est mise en oeuvre en discontinu, la quantité de formiate de méthyle est maintenue inférieure aux valeurs indiquées bien qu'évoluant en décroissant, car ledit ester est consommé par la réaction. Quant à la pression partielle en monoxyde de carbone, elle peut ou non être maintenue constante, à la condition de se trouver dans la plage de valeurs indiquées.

Les deux caractéristiques qui viennent d'être explicitées sont essentielles pour obtenir un procédé dont la productivité est considérablement améliorée.

Le procédé d'isomérisation selon l'invention est mis en oeuvre en présence d'eau. La quantité d'eau, exprimée en poids par rapport au mélange réactionnel, varie entre 0 exclu et 5 %. D'une manière avantageuse, ladite teneur est comprise entre 0 exclu et 2 % en poids.

II est à noter que l'eau joue un rôle important dans le procédé. En effet, celle-ci participe au maintien en solution du catalyseur, en particulier dans la zone de vaporisation partielle du mélange (flash) qui sera décrite ultérieurement. Elle permet aussi de limiter les réactions secondaires connues pour les procédés mis en oeuvre dans des conditions anhydres.

Par ailleurs, la quantité de promoteur halogéné maintenue pendant la réaction est plus particulièrement comprise entre 0,1 et 20 % en poids du mélange réactionnel. De préférence, la teneur en promoteur halogéné est comprise entre 1 et 15 % en poids du mélange réactionnel.

Il est à noter que les quantités de promoteur indiquées ci-dessus sont données à titre indicatif. En effet, l'homme du métier est à même de trouver le compromis optimal entre, d'une part, une efficacité maximale de ce composé, qui a un effet bénéfique sur l'activité et la stabilité du catalyseur, et d'autre part, des considérations économiques liées au coût entraîné dans le procédé par le recyclage de ce composé.

Le complément à 100 % est constitué par le solvant de la réaction. Plus particulièrement, ce dernier comprend l'acide produit, éventuellement l'ester produit, et de l'acide formique.

Selon une variante particulière de l'invention, la quantité d'acide formique présent dans le milieu réactionnel est maintenue inférieure à 15 % en poids du mélange réactionnel. De préférence, la teneur en acide formique est maintenue inférieure à 12 % et plus particulièrement inférieure à 10 % en poids du mélange réactionnel.

Par ailleurs, selon un mode de réalisation avantageux de la présente invention, la quantité d'acides carboxyliques libres présents dans le mélange réactionnel est supérieure à 25 % en poids dudit mélange et telle que la totalité des constituants du mélange réactionnel représente 100 % en poids du mélange réactionnel. Plus particulièrement, la quantité en acides carboxyliques libres est supérieure à 30 % en poids du mélange réactionnel, et de préférence, elle est supérieure à 40 % en poids du mélange réactionnel.

Dans le cas où le co-solvant est présent, de préférence l'acétate de méthyle, la teneur pondérale de celui-ci est de préférence inférieure ou égale à celle de l'acide acétique.

Il est à noter que le rapport molaire acide formique /acétate de méthyle peut être différent de 1 dans les conditions de la réaction, c'est-à-dire supérieur ou inférieur à cette valeur. On peut bien évidemment effectuer la réaction avec un rapport molaire égal à 1.

Généralement, la concentration totale en iridium dans le milieu réactionnel est comprise entre 0,1 et 100 mmol/l de préférence entre 1 et 25 mmol/l,

La réaction d'isomérisation faisant l'objet de l'invention est de préférence mise en oeuvre en présence d'une teneur en métaux de corrosion inférieure à 2000 ppm. Les métaux de corrosion sont notamment le fer, le nickel, le chrome, le molybdène. La teneur en métaux de corrosion dans le mélange réactionnel est maintenue par les méthodes connues de l'homme du métier, comme par exemple la précipitation sélective, l'extraction liquide liquide, le passage sur des résines échangeuses d'ions.

La réaction est en général mise en oeuvre à une température comprise entre 150 et 250°C. Plus particulièrement la température de réaction est comprise entre 175 et 210°C. De préférence, elle est comprise entre 175 et 200°C.

La pression totale sous laquelle est conduite la réaction est en général supérieure à la pression atmosphérique. Plus particulièrement elle est inférieure à 100.10⁵ Pa et de préférence inférieure ou égale à 50.10⁵ Pa. Les pressions sont exprimées en pascals absolus, et ont été mesurées à chaud, c'est-à-dire dans les conditions de température de la réaction.

La réaction est mise en oeuvre dans des appareillages résistant à la corrosion créée par le milieu. Ainsi, le zirconium ou encore les alliages du type Hastelloy® C ou B, conviennent particulièrement bien aux conditions de mise en oeuvre de la réaction.

Lors du démarrage de la réaction, les divers composants sont introduits dans un réacteur approprié, muni de moyens d'agitation pour assurer une bonne homogénéité du mélange réactionnel. Il est à noter que si le réacteur comprend de préférence des moyens d'agitation mécanique du mélange réactionnel, il n'est pas exclu d'opérer sans de tels moyens, l'homogénéisation du mélange pouvant être réalisée par l'introduction du monoxyde de carbone dans le réacteur.

Il est à noter que la réaction pourrait être convenablement mise en oeuvre dans un réacteur de type piston.

La combinaison de plusieurs réacteurs de type agité et piston est bien entendu envisageable.

Le mélange réactionnel en sortie du réacteur est traité de manière appropriée pour séparer les produits du mélange réactionnel comprenant notamment le catalyseur.

A ce titre, et dans le cas de mise en oeuvre de la réaction en continu, on peut employer par exemple une technique classique consistant à détendre le mélange de manière à provoquer une vaporisation partielle de ce dernier. Cette opération peut être mise en oeuvre grâce à une vanne permettant de détendre le mélange, ce dernier étant introduit dans un séparateur (dit séparateur flash). L'opération peut avoir lieu avec ou, de préférence, sans apport de chaleur, c'est-à-dire dans des conditions adiabatiques.

Selon une variante de l'invention, la teneur en eau dans la zone de vaporisation partielle est maintenue pour la partie non vaporisée, à une valeur d'au moins 0,5 % en poids par rapport au poids de cette partie non vaporisée. Ceci peut avoir lieu, si nécessaire, par injection d'eau dans ladite zone de vaporisation partielle, c'est-à-dire dans le séparateur flash.

La partie vaporisée, comprenant l'acide acétique et/ou l'acétate de méthyle produits, peut être mise en contact et lavée dans la partie supérieure du séparateur flash par un liquide provenant des installations de purification en aval.

En sortie du séparateur flash, la partie non vaporisée comprenant notamment le catalyseur resté en solution, pour tout ou partie, est recyclée de manière avantageuse au réacteur, classiquement au moyen d'une pompe.

La partie vaporisée, comprenant l'acide acétique et/ou l'acétate de méthyle produits, est ensuite envoyée dans une zone de purification comprenant, de manière habituelle, diverses colonnes de distillation.

Selon une variante de l'invention, une mise en contact et un lavage complémentaires peuvent être réalisés dans la première colonne de distillation à l'aide d'un liquide provenant des installations de purification.

Selon une autre variante de l'invention, le mélange réactionnel en sortie du réacteur peut être directement détendu dans la première colonne de distillation de la zone de purification.

Les différents flux de matière séparés dans la zone de purification peuvent être recyclés vers le réacteur ou traités de façon indépendante.

L'acide acétique ou l'acétate de méthyle obtenu par ce procédé est de qualité suffisante pour être vendu sans purification autre que celles connues de l'homme de l'art et déjà dans le domaine public.

L'introduction de monoxyde de carbone peut avoir lieu directement dans le réacteur, mais aussi dans la zone de recyclage de la fraction liquide non vaporisée, de telle sorte que le monoxyde de carbone ne soit pas dégazé directement vers la zone de vaporisation partielle du mélange réactionnel. Dans ce but, l'introduction du monoxyde de carbone selon cette dernière possibilité, est plus particulièrement effectuée en aval de la pompe de recyclage du mélange réactionnel.

Un exemple concret mais non limitatif de l'invention va maintenant être présenté.

### EXEMPLE

Tout d'abord, la solution catalytique est préparée comme suit :
On introduit dans un autoclave :
   * 105 g d'iodure d'iridium,
   * 90 g d'acide iodhydrique en solution à 57 % dans l'eau
   * 910 g d'acide acétique

L'autoclave est ensuite pressurisé avec 50.10⁵ Pa (50 bar) de monoxyde de carbone.

On porte la température à 150°C.

La durée de la réaction est de 4 heures.

L'autoclave est ensuite dépressurisé puis le milieu réactionnel refroidi.

On obtient une solution de couleur rouge, que l'on décante pour obtenir la solution catalytique.

La réaction d'isomérisation est mise oeuvre comme suit :
Dans un autoclave en Hastelloy® B2, on introduit en continu une solution d'iridium dans l'acide acétique, préparée comme ci-dessus, de l'acide acétique, de l'iodure de méthyle, du formiate de méthyle et de l'eau.

La pression partielle en monoxyde de carbone est maintenue constante à une valeur de 6.10⁵ Pa (6 bar).

La pression totale en sortie du réacteur est de 25.10⁵ Pa (25 bar).

La température est maintenue à 190 °C ± 0,5°C.

### La composition du mélange réactionnel en régime stabilisé est la suivante:

| | |
|---|---|
| eau | 0,20 % |
| acétate de méthyle | 7,8 % |
| iodure de méthyle | 12 % |
| acide formique | 3,7 % |
| formiate de méthyle | 0,72 % |
| la concentration en iridium est de | 3080 ppm |
| acide acétique | complément à 100 % |

La composition du mélange réactionnel, en pourcent massique, donnée avec une précision de l'ordre de 2%, est déterminée par dosage en chromatographie en phase vapeur.

Le calcul de la vitesse d'isomérisation est effectué sur le liquide issu du réacteur, refroidi à température ambiante et, collecté pendant une durée comprise entre 30 et 60 minutes par rapport aux flux des composés injectés dans le réacteur pendant cette même durée, après que le régime chimique stabilisé a été obtenu.

On obtient une vitesse d'isomérisation de 25 mol/h/l en acide acétique formé, ce dernier étant sous la forme d'acide et d'acétate de méthyle.

La proportion entre ces deux produits est la suivante : 75 % d'acide acétique et 25 % d'acétate de méthyle.

Les seuls composés détectés lors de l'analyse du mélange réactionnel sont l'iodure de méthyle, l'acide formique, le formiate de méthyle, l'acétate de méthyle, l'eau et l'acide acétique.

## Revendications

1. Procédé de préparation d'acide acétique et/ou d'acétate de méthyle par réaction d'isomérisation du formiate de méthyle, en présence d'eau, d'un solvant et d'un système catalytique comprenant au moins un promoteur halogèné et au moins un composé à base d'iridium, **caractérisé en ce que** ledit procédé est un procédé continu dans lequel on maintient une pression partielle en monoxyde de carbone comprise entre 0,1.10⁵ Pa et 25.10⁵ Pa, une quantité de formiate de méthyle inférieure à 20 % en poids du mélange réactionnel et une quantité d'eau comprise entre 0 exclu et 5% en poids du mélange réactionnel.

2. Procédé selon la revendication 1, **caractérisé en ce que** la réaction est mise en oeuvre en maintenant une pression partielle en monoxyde de carbone supérieure ou égale à 0,5.10⁵ Pa, de préférence supérieure ou égale à 10⁵ Pa.

3. Procédé selon l'une des revendications 1 à 2, **caractérisé en ce que** la réaction est mise en oeuvre en maintenant une pression partielle en monoxyde de carbone inférieure ou égale à 15.10⁵ Pa, de préférence inférieure ou égale à 10.10⁵ Pa.

4. Procédé selon l'une des revendications 1 à 3, **caractérisé en ce que** l'on maintient une quantité d'eau comprise entre 0 exclu et 2 % en poids du mélange réactionnel.

5. Procédé selon l'une des revendications 1 à 4, **caractérisé en ce que** la réaction est mise en oeuvre en maintenant une quantité de formiate de méthyle inférieure à 10 % en poids du mélange réactionnel, de préférence inférieure à 5 % en poids du mélange réactionnel.

6. Procédé selon l'une des revendications 1 à 5, **caractérisé en ce que** la réaction est mise en oeuvre en maintenant une quantité de promoteur halogéné comprise entre 0,1 et 20 % en poids du mélange réactionnel, de préférence comprise entre 1 et 15 % en poids du mélange réactionnel.

7. Procédé selon l'une des revendications 1 à 6, **caractérisé en ce que** la réaction est mise en oeuvre en présence d'un solvant qui est choisi parmi les acides carboxyliques aliphatiques comportant 2 à 10 atomes de carbone.

8. Procédé selon l'une des revendications 1 à 7, **caractérisé en ce que** la réaction est mise en oeuvre en présence d'acide formique à une teneur maintenue inférieure à 15 % en poids du mélange réactionnel, et de préférence inférieure à 12 % en poids du mélange réactionnel.

9. Procédé selon la revendication 8, **caractérisé en ce que** la teneur en acide formique est maintenue inférieure à 10 % en poids du mélange réactionnel.

10. Procédé selon l'une des revendications 7 à 9, **caractérisé en ce que** la quantité d'acides carboxyliques libres présents dans le mélange réactionnel est supérieure à 25 % en poids dudit mélange et telle que la totalité des constituants du mélange réactionnel représente 100 % en poids du mélange réactionnel.

11. Procédé selon l'une des revendications 1 à 10, **caractérisé en ce que** la réaction est mise en oeuvre en présence d'un co-solvant qui est l'acétate de méthyle.

12. Procédé selon la revendication 11, **caractérisé en ce que** la teneur pondérale en co-solvant est inférieure ou égale à celle de l'acide acétique.

13. Procédé selon l'une des revendications 1 à 12, **caractérisé en ce que** la réaction est mise en oeuvre en présence d'un promoteur halogéné choisi parmi les composés iodés ou un précurseur de tels composés.

## Patentansprüche

1. Verfahren zur Herstellung von Essigsäure und/oder Methylacetat durch Isomerisierung von Methylformiat in Gegenwart von Wasser, eines Lösungsmittels und eines katalytischen Systems, das mindestens einen halogenierten Promotor und mindestens eine Verbindung auf Iridiumbasis umfasst, **dadurch gekennzeichnet, dass** das genannte Verfahren ein kontinuierliches Verfahren ist, bei dem man einen Kohlenmonoxid-Partialdruck zwischen 0,1.10⁵ und 25.10⁵ Pa und eine Methylformiat-Menge von weniger als 20 Gew.-%, bezogen auf das Gewicht der Reaktionsmischung, und eine Wassermenge zwischen > 0 und 5 Gew.-%, bezogen auf das Gewicht der Reaktionsmischung, aufrecht erhält.

2. Verfahren nach Anspruch 1, **dadurch gekennzeichnet, dass** man die Reaktion durchführt, indem man einen Kohlenmonoxid-Partialdruck von ≥ 0,5.10⁵ Pa, vorzugsweise von ≥ 10⁵ Pa, aufrecht erhält.

3. Verfahren nach einem der Ansprüche 1 bis 2, **dadurch gekennzeichnet, dass** man die Reaktion durchführt, indem man einen Kohlenmonoxid-Partialdruck von ≤ 15.10⁵ Pa, vorzugsweise von ≤ 10.10⁵ Pa, aufrecht erhält.

4. Verfahren nach einem der Ansprüche 1 bis 3, **dadurch gekennzeichnet, dass** man eine Wassermenge zwischen > 0 und 2 Gew.-%, bezogen auf das Gewicht der Reaktionsmischung, aufrecht erhält.

5. Verfahren nach einem der Ansprüche 1 bis 4, **dadurch gekennzeichnet, dass** man die Reaktion durchführt, indem man eine Methylformiatmenge von < 10 Gew.-%, bezogen auf das Gewicht der Reaktionsmischung, vorzugsweise von < 5 Gew.-%, bezogen auf das Gewicht der Reaktionsmischung, aufrecht erhält.

6. Verfahren nach einem der Ansprüche 1 bis 5, **dadurch gekennzeichnet, dass** man die Reaktion durchführt, indem man eine Menge an halogeniertem Promotor zwischen 0,1 und 20 Gew.-%, bezogen auf das Gewicht der Reaktionsmischung, vorzugsweise zwischen 1 und 15 Gew.-%, bezogen auf das Gewicht der Reaktionsmischung, aufrecht erhält.

7. Verfahren nach einem der Ansprüche 1 bis 6, **dadurch gekennzeichnet, dass** man die Reaktion in Gegenwart eines Lösungsmittels durchführt, das ausgewählt wird unter den aliphatischen Carbonsäuren mit 2 bis 10 Kohlenstoffatomen.

8. Verfahren nach einem der Ansprüche 1 bis 7, **dadurch gekennzeichnet, dass** man die Reaktion durchführt in Gegenwart von Ameisensäure, deren Gehalt bei weniger als 15 Gew.-%, bezogen auf das Gewicht der Reaktionsmischung, vorzugsweise bei weniger als 12 Gew.-%, bezogen auf das Gewicht der Reaktionsmischung, gehalten wird.

9. Verfahren nach Anspruch 8, **dadurch gekennzeichnet, dass** der Gehalt an Ameisensäure bei weniger als 10 Gew.-%, bezogen auf das Gewicht der Reaktionsmischung, gehalten wird.

10. Verfahren nach einem der Ansprüche 7 bis 9, **dadurch gekennzeichnet, dass** die Menge der freien Carbonsäuren, die in der Reaktionsmischung vorliegen, mehr als 25 Gew.-%, bezogen auf das Gewicht der Reaktionsmischung, beträgt und so groß ist, dass die Gesamtmenge der Bestandteile der Reaktionsmischung 100 Gew.-% der Reaktionsmischung darstellt.

11. Verfahren nach einem der Ansprüche 1 bis 10, **dadurch gekennzeichnet, dass** man die Reaktion in Gegenwart eines Colösungsmittels, bei dem es sich um Methylacetat handelt, durchführt.

12. Verfahren nach Anspruch 11, **dadurch gekennzeichnet, dass** der Gewichtsgehalt an Colösungsmittel kleiner als oder gleich demjenigen an Essigsäure ist.

13. Verfahren nach einem der Ansprüche 1 bis 12, **dadurch gekennzeichnet, dass** man die Reaktion in Gegenwart eines halogenierten Promotors durchführt, der ausgewählt wird aus den iodierten Verbindungen oder einem Vorläufer dieser Verbindungen.

## Claims

1. Method of preparing acetic acid and/or methyl acetate by methyl formate isomerisation, in the presence of water, a solvent and a catalytic system comprising at least one halogenated promoter and at least one iridium-based compound, **characterised in that** said method is a continuous method wherein a partial carbon monoxide pressure is maintained between 0.1 . 10⁵ Pa and 25 . 10⁵ Pa, an amount of methyl formate is maintained below 20% by weight of the reaction mixture and a water content comprised between O (excluded) and 5 % by weight of the reaction mixture is maintained.

2. Method according to claim 1, **characterised in that** the reaction is carried out in maintaining a partial carbon monoxide pressure greater than or equal to 0.5 . 10⁵ Pa, preferably greater than or equal to 10⁵ Pa.

3. Method according to one of claims 1 to 2, **characterised in that** the reaction is carried out in maintaining a partial carbon monoxide pressure lower than or equal to 15 . 10⁵ Pa, preferably lower than or equal to 10 . 10⁵ Pa.

4. Method according to one of claims 1 to 3, **characterised in that** an amount of water between 0 (excluded) and 2% by weight of the reaction mixture is maintained.

5. Method according to one of claims 1 to 4, **characterised in that** the reaction is carried out in maintaining an amount of methyl formate lower than 10% by weight of the reaction mixture, preferably lower than 5% by weight of the reaction mixture.

6. Method according to one of claims 1 to 5, **characterised in that** the reaction is carried out in maintaining an amount of halogenated promoter between 0.1 and 20% by weight of the reaction mixture, preferably between 1 and 15% by weight of the reaction mixture.

7. Method according to one of claims 1 to 6, **characterised in that** the reaction is carried out in the presence of a solvent which is selected from the aliphatic carboxylic acids having 2 to 10 carbon atoms.

8. Method according to one of claims 1 to 7, **characterised in that** the reaction is carried out in the presence of formic acid at a content maintained below 15% by weight of the reaction mixture, and preferably below 12% by weight of the reaction mixture.

9. Method according to claim 8, **characterised in that** the formic acid content is maintained below 10% by weight of the reaction mixture.

10. Method according to one of claims 7 to 9, **characterised in that** the amount of free carboxylic acids present in the reaction mixture is greater than 25% by weight of said mixture and such that the whole of the constituents of the reaction mixture represents 100% by weight of the reaction mixture.

11. Method according to one of claims 1 to 10, **characterised in that** the reaction is carried out in the presence of a co-solvent which is methyl acetate.

12. Method according to claim 11, **characterised in that** the weight content of the co-solvent is lower than or equal to that of acetic acid.

13. Method according to one of claims 1 to 12, **characterised in that** the reaction is carried out in the presence of a halogenated promoter selected from iodinated compounds or a precursor of such compounds.
